Europäisches Patentamt

European Patent Office

Office européen des brevets

(19)

(11) Publication number: **0 072 607**
A2

# (12) EUROPEAN PATENT APPLICATION

(21) Application number: **82301998.9**

(22) Date of filing: **19.04.82**

(51) Int. Cl.³: **A 61 F 5/44**
**A 61 L 15/06**

(30) Priority: **20.04.81 US 255402**
**05.03.82 US 355070**

(43) Date of publication of application:
**23.02.83 Bulletin 83/8**

(84) Designated Contracting States:
**DE FR GB IT**

(71) Applicant: **PFIZER INC.**
**235 East 42nd Street**
**New York, N.Y. 10017(US)**

(72) Inventor: **Bauer, Stuart Michael**
**76 Toby Drive**
**Succasunna New Jersey(US)**

(74) Representative: **Graham, Philip Colin Christison et al,**
**Pfizer Limited Ramsgate Road**
**Sandwich, Kent CT13 9NJ(GB)**

(54) Adhesive pad for ostomy use.

(57) An adhesive pad suitable for use by ostomates comprising a water permeable non-woven fabric backing having on one surface thereof an adhesive composition comprising an essentially homogeneous mixture of vinyl acetate copolymer and a water swellable hydrogel.

EP 0 072 607 A2

**0072607**

P.C. 6404/A

## ADHESIVE PAD FOR OSTOMY USE

This invention relates to novel pressure-sensitive adhesive pads suitable for prolonged use on both moist and dry skin. More particularly, this invention relates to stomal adhesive pads.

A stomal adhesive pad, which affixes to the skin a collection bag necessitated by gastrointestinal surgery, must adhere to both moist and dry skin for prolonged periods of time, and in addition· must be non-irritating, adhere securely during use yet be easily removable after use, must accomodate skin perspiration, form a barrier to stomal leakage, be adaptable to the size requirements of individual ostomates, and at the same time be economically and technically feasible to manufacture, package and store.

A bandage for both moist and dry skin is disclosed by Chen in U.S. Patent No. 3,339,546. This bandage has been adapted for ostomy use, but employs a polymeric film backing which is water impervious and therefore traps moisture exuded by the underlying skin. The entrapped moisture causes weakening of the adhesive and as a result the pad "floats off". A further disadvantage of this polymeric film backing is its difficulty of handling during the manufacturing process. It is delicate, has low machine strength, easily wrinkles and is difficult to laminate.

An improved ostomy adhesive pad disclosed by Chen et al. in U.S. Patent No. 4,253,460 comprises a continuous or discontinuous film backing. Although the discontinuities improve the breathability of the backing, the film still offers manufacturing problems.

Backings other than polymeric film have been suggested by Maeth in U.S. Patent No. 3,249,109 who discloses woven fabrics such as muslin and polyester, and Eustis et al. in U.S. Patent No. 2,429,223 who disclose polyvinyl ester fabric. These backings have limited ostomy related utility where a barrier to matter extrinsic to the skin is necessary.

The adhesive compositions in stomal pads must withstand prolonged exposure to skin moisture while retaining adherency to the skin. Chen in U.S. Patent 3,339,546, cited above, suggests a mixture of hydrocolloid and a viscous elastic binder, preferably polyisobutylene. Chen et al. in U.S. Patent No. 4,253,450, cited above, disclose a mixture of various hydrocolloid gums, pressure sensitive adhesives and cohesive strengthening agents. Marsan in U.S. Patent 3,908,658 discloses a mineral-oil based product comprising an ethylene-vinyl acetate copolymer and styrene-isobutylene copolymer.

It has now been discovered, however, that a simple combination of a water-swellable hydrogel, and an elastic non-flowing adhesive carrier on a non-woven fabric backing comprises an adhesive pad with excellent properties for use as a stomal adhesive pad.

It is a main object of this invention to provide an adhesive pad for use by ostomates which is capable of adhering to both moist and dry skin surfaces for prolonged periods of time.

It is a further object of the present invention to provide an adhesive pad which is non-irritating to the skin while providing a barrier to irritating matter extraneous to the skin such as leakage of stomal discharge.

A further object of the present invention is to provide an adhesive pad which may be used to secure a collection bag to a stoma of any size.

Another object of the present invention is to provide an adhesive pad which is economically feasible to manufacture, package and store.

These and other objects of the invention are achieved with a novel adhesive pad comprising a water permeable non-woven fabric backing having on one surface thereof an adhesive composition comprising an essentially homogeneous mixture of a vinyl acetate copolymer carrier and a water-swellable hydrogel, said hydrogel being present in an amount sufficient to enable the pad to adhere to moist body surfaces.

FIG. 1 is a pictorial view of the adhesive pad of the present invention.

Fig. 2 is a pictorial view of the adhesive pad of the present invention having affixed thereto a connection means for a collection bag (not shown).

The invention will be described in detail with reference to the preferred embodiment thereof, which is the combination of a non-woven polyester fabric mat and affixed to one surface thereof an adhesive composition comprised of a mixture of a starch graft acrylamide sodium acrylate terpolymer hydrogel and vinyl acetate copolymer carrier.

Reference to this embodiment does not limit the scope of the invention, which is limited only by the scope of the claims.

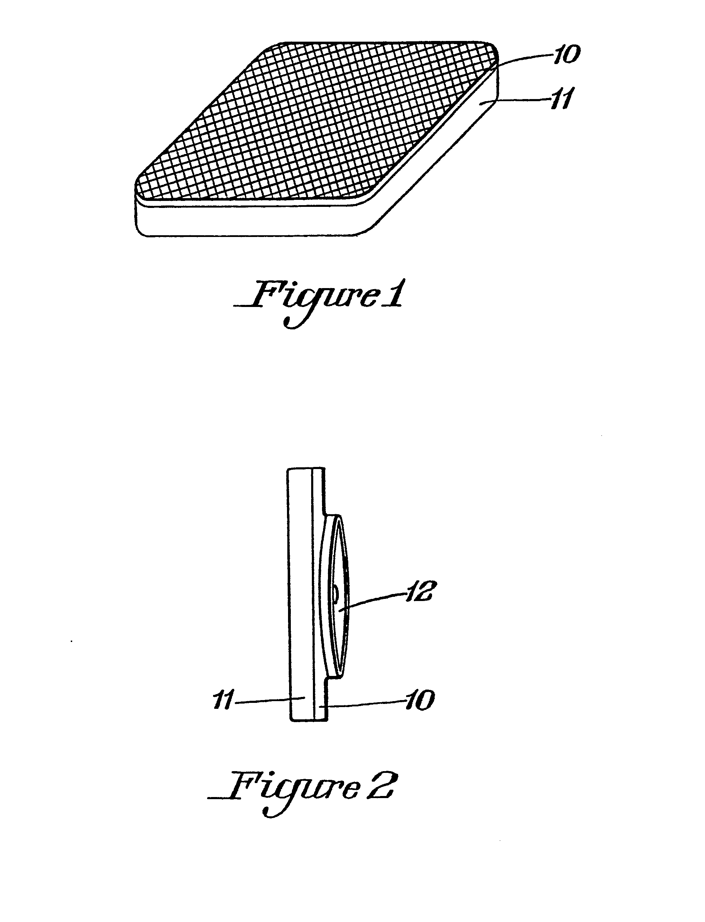

A preferred embodiment of the present invention is shown in FIG. 1. A non-woven fabric mat (10) having deposited on one side thereof adhesive composition (11) is shown.

Non-woven fabric mat (10) is preferably a polyester fiber of the type disclosed in Canadian Patent No. 3,542,634 as manufactured for example by Kendall Company. This non-woven fabric is manufactured by a compression process which produces a mesh work of fibers defining a porous structure. These pores become the means by which perspiration evaporates without being trapped when the adhesive pad is applied to the skin. The skin thus remains comfortable beneath the pad and the useful adherent lifetime of the pad is extended. The strength of this non-woven polyester fiber is sufficient to make it easily machinable which makes it convenient to use for manufacture of adhesive pads.

Adhesive composition (11) is an intimate mixture of a water-swellable hydrogel and an elastomeric carrier. The preferred hydrogel is a starch graft acrylamide sodium acrylate terpolymer for example of the type manufactured by Henkel corporation under the tradename of SGP Absorbent Polymer. Water absorption by the starch graft copolymer is reversible so that when water evaporates from the swollen hydrogel, the polymer returns to its original state. Moreover dried polymer will reswell when water is admitted allowing repeated use and prolonging the adherent lifetime of the adhesive pad.

The preferred elastomeric carrier is a vinyl acetate copolymer such as the product manufactured by Air Products under the tradename Flexbond. Preferably the vinyl acetate copolymer is a mixture of a vinyl acetate-dioctylmaleate emulsion and a vinyl acetate ethylene emulsion. This non-flowing elastomer provides dry adhesion and support for the hydrogel and ensures that the adhesive will not ooze during storage.

The novel adhesive pad of the present invention is generally prepared by first adding the water-swellable hydrogel to a quantity of water sufficient to fully hydrate the dry powder. This is followed by the addition of the vinyl acetate copolymer and the subsequent homogenization and deaerating of the resultant blend using equipment commonly used for this purpose. The adhesive composition is comprised of 70% to 99% vinyl acetate copolymer and 1% to 30% water swellable hydrogel. Preferably the adhesive composition is comprised of about 90% vinyl acetate copolymer and about 10% swellable hydrogel. Most preferably the vinyl acetate copolymer is comprised of 90% to 75% vinyl acetate - dioctylmaleate and 10% to 25% of vinyl acetate-ethylene. This smooth air-bubble-free material is then knife coated onto the water-permeable non-woven fabric backing and this is then allowed to dry. The process may be repeated a sufficient number of times to build up layers of adhesive to make a maleable but semi-rigid adhesive pad. Finally a suitable silicone treated release paper is applied to the adhesive surface. Finished product may then be die cut and packaged for final use.

A connective means for collection bags may be fixed to the exposed backing of the adhesive pad as shown in FIG. 2. In the preferred embodiment of the invention, the connecting means is a circular plastic ring designed to snap on to the mouth of the collection bag to form a water-tight and odor-tight seal. The central hole may be enlarged by the individual ostomate to accomodate a stoma of any size.

Medicaments such as antibiotics, emollients, acne aids, etc. can be incorporated in the adhesive mixture if it is desired to treat the skin with these medicaments while the adhesive pad is applied thereto.

CLAIMS

1. An adhesive pad comprising a water permeable non-woven fabric backing having on one surface thereof an adhesive composition comprising an essentially homogeneous mixture of vinyl acetate copolymer and a water-swellable hydrogel said hydrogel being present in an amount sufficient to enable the pad to adhere to moist body surfaces.

2. An adhesive pad as claimed in claim 1 wherein said non-woven fabric backing is polyester and said adhesive composition comprises 70% to 99% vinyl acetate copolymer and 1% to 30% water swellable hydrogel.

3. An adhesive pad as claimed in claim 2 wherein said vinyl acetate copolymer comprises vinyl acetate-dioctyl maleate.

4. An adhesive pad as claimed in claim 2 wherein said vinyl acetate copolymer comprises vinyl acetate-dioctyl maleate and vinyl acetate-ethylene.

5. An adhesive pad as claimed in claim 2 wherein said adhesive composition comprises about 70% vinyl acetate – dioctyl maleate copolymer, about 20% vinyl acetate-ethylene copolymer and about 10% water swellable starch graft acrylamide acrylate terpolymer.

6. An ostomy device comprising the adhesive pad of claim 1 and affixed thereto a connector adapted to receive a suitable collection receptacle.

0072607

Figure 1

Figure 2